# EUROPEAN PATENT SPECIFICATION

(11) **EP 0 503 031 B1**
(45) Date of publication and mention of the grant of the patent: **01.04.1998**
(21) Application number: 91917125.6
(22) Date of filing: 26.09.1991
(51) Int. Cl.: A61M 15/00

(54) **INHALER DEVICES PROVIDED WITH A RESERVOIR FOR SEVERAL DOSES OF MEDIUM FOR INHALING, TRANSPORTING DEVICE, WHIRL CHAMBER**
INHALATOR MIT EINEM RESERVOIR FÜR MEHRERE DOSIERTE INHALATIONSMITTEL, MIT TRANSPORTVORRICHTUNG UND MIT WIRBELKAMMER
DISPOSITIF D'INHALATION POURVU D'UN RESERVOIR POUR PLUSIEURS DOSES DE PRODUIT D'INHALATION, DISPOSITIF DE TRANSPORT, CHAMBRE DE TURBULENCE

(30) Priority: 26.09.1990 NL 9002103; 15.07.1991 NL 9101245; 20.09.1991 NL 9101593
(43) Date of publication of application: 16.09.1992
(60) Divisional application: 94203256.6
(73) Proprietor: PHARMACHEMIE B.V., 2031 GA Haarlem (NL); RAYTEC B.V., 5253 CA Nieuwkuijk (NL)
(72) Inventor: ZANEN, Pieter, NL-3993 RH Houten (NL); PLOMP, Adrianus, NL-1871 TJ Schoorl (NL); BOON, Gerhardus, Anthonius, NL-1531 LC Wormer (NL); VAN SWIETEN, Roy, NL-5253 AP Nieuwkuyk (NL)
(74) Representative: Land, Addick Adrianus Gosling
(86) International application number: EP9101884
(87) International publication number: WO9204928

(56) References cited:
- DE-A- 4 004 904
- FR-A- 2 347 939
- FR-A- 2 489 116
- FR-A- 2 598 918
- GB-A- 1 118 341
- GB-A- 2 165 159
- US-A- 1 459 478
- US-A- 2 581 182
- US-A- 4 274 403

## Description

The present invention relates to inhaler devices, in particular inhaler devices with which several doses can be administered. With such inhaler devices an aerosol is administered as medicine to the lungs, particularly the alveolar zone, by inhalation through nose or mouth.

Existing systems typically provide a single dose of an aerosol, whereafter a capsule is thrown away, which is harmful to the environment. Inhaler devices, for instance for CNSLD patients, wherein at each inhalation a capsule is placed therein, are frequently used in practice.

Inhaler devices for several doses are still only commercially available on a limited scale. There are problems with these known inhaler devices in respect of their manageability and size, the required suction force on a mouth or nose piece and/or the accuracy of dosing.

Known inhaler devices for several doses are awkward to use and/or consist of a relatively large number of components, whereby manufacture becomes time-consuming and expensive. Such an inhaler device is known from EP-A-211595.

Further in US patent US-A-4,274,403 an inhaler is shown, including a dispensing cylinder contained in a transfer bore in a dispensing head. This known inhaler device has a relatively large number of parts. Also the dispensing cylinder extends beyond the housing of the inhaler itself, while accurate dispensing is not secure because of the transfer direction of the cylinder.

The present invention provides a device for inhaling an aerosol, comprising:
- a housing in which a reservoir for powder is received;
- an inhaling piece on the housing; and
- transporting means for transporting the powder from the reservoir to the inhaling piece in a dose required for the aerosol, said transporting means including a plunger which is provided with a recess, and which is movable reciprocally counter to spring pressure, characterized by compressing means for holding the content of the reservoir in compressed state and in that said plunger is movable axially relative to said reservoir, between a first axial position wherein said recess is inside said reservoir and a second axial position wherein said recess is positioned outside of said reservoir and is in communication with said inhaling piece.

The present invention provides an inhaler device that can be used in different positions. The operation of the inhaling piece, the transporting means and/or the whirl chamber are not adversely affected by the position of the inhaler device relative to the direction of the force of gravity.

The whirling and high speed of the air are advantageous in obtaining particles for inhaling of a sufficiently small diameter (for instance smaller than 7, 5 or 1 µm). The medium in the reservoir usually also comprises carrier material to cause the medium to flow easily, particularly from the reservoir to the whirl chamber where the particles for inhaling are then separated from the carrier particles (of usually greater diameter) by the shear forces generated by the air. Also in the case the medium of inhaling does not comprise any carrier material, shear forces have to be applied thereto, since such inhaling medium is composed of complexes of relatively large diameter, for instance greater than 7 µm. For inhalation into the alveolar zone the particle diameter also has to be reduced. With both types of medium there normally remains a non-inhalable fraction of for example 20% by vol.

Further preferred embodiments relate to the desired whirling of the air and the compactness of the inhaler device; such embodiments are specified in claim 2 - 4.

In preference the inhaler device comprises two or more mutually interchangeable reservoirs with different medium. A patient then only needs to carry one inhaler device on his/her person, even when (s)he has to inhale different types of medium. Such reservoirs can be provided in simple manner with a bayonet fitting for coupling to the remaining part of the inhaler device according to the present invention.

Inhaler devices provided with a mixing chamber, wherein at each inhalation a capsule with a dose of medium is introduced, in addition to a reservoir for several doses of medium for inhaling are widely available, for instance for CNSLD patients. The medium for inhaling typically comprises a powder which during inhalation must be present in the air in a particular diameter of for instance less than 10µm, or less than 7, 5, or 1µm. To improve the transporting properties of the powder, carrier material can be incorporated therein with a particle diameter greater than the above mentioned diameter. Also when no carrier material is included in powder, complexes of particles occur easily in the aerosol which have a diameter greater than above stated. Particles with a greater diameter than the above states are undesirable in the aerosol as they cannot exert any medicinal effect but can still result in irritations of the bronchial tubes, oral cavity and/or pharynx.

Further advantages, features and details of the present invention will be elucidated in the light of a description of preferred embodiments thereof with reference to the annexed drawing, in which:
Fig. 1 shows a view, partly in section and partly in perspective, of a preferred embodiment according to the present invention in closed situation;
Fig. 2 is the view of fig. 1 in the ready-for-use situation;
Fig. 3 shows a view, partly in section and partly in perspective, of the embodiment of fig. 1 and 2 for elucidation of several details thereof;
Fig. 4 shows a view of an element to be included in the embodiment of fig. 1-3;
Fig. 5 shows, for illustrative purposes, a partly broken away view in perspective of an inhaler device not according to the present invention;
Fig. 6 is a view, partly in perspective and partly in section, of detail II of fig. 5; and
Fig. 7A and 7B respectively show elements which can be incorporated in the device as shown in fig. 5 and 6; and
Fig. 8 shows, again for illustrative purposes, a side view of an inhaler device partly in section, also not according to the present invention.

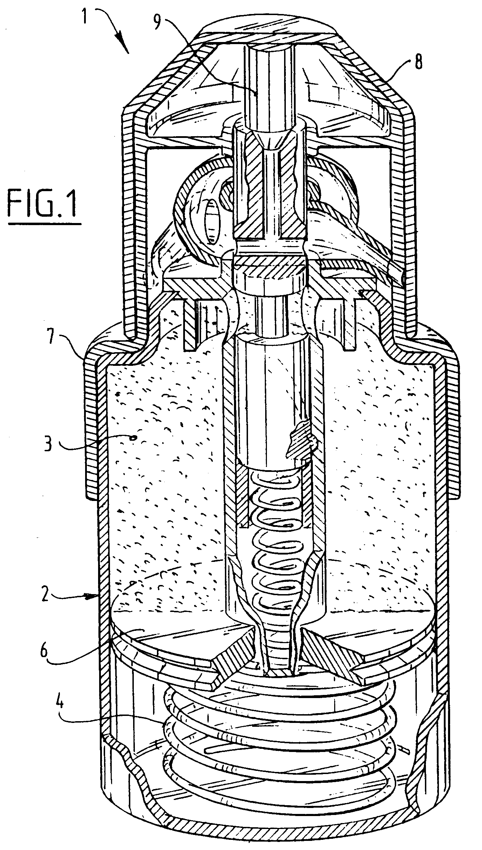

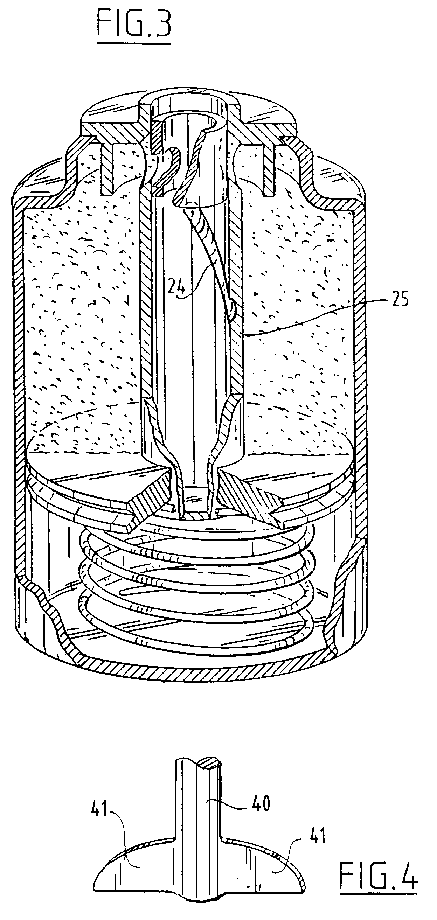

An inhaler device 1 (fig. 1-3) according to the present invention comprises a housing 2 in which a reservoir 3 of inhalable material, which is medicinal in the embodiment shown, is held in a compressed state. Placed on the housing 2 is an inhaling piece 7, for example joined thereto in a manner not shown with a snap-on connection, so that after the reservoir has been emptied the inhaling piece can be placed on a new reservoir. The inhaling piece 7 is intended for placing on nose or mouth in order to be then sucked on. Placed over the inhaling piece 7 is a removable closing cap 8 provided with a protruding pin 9.

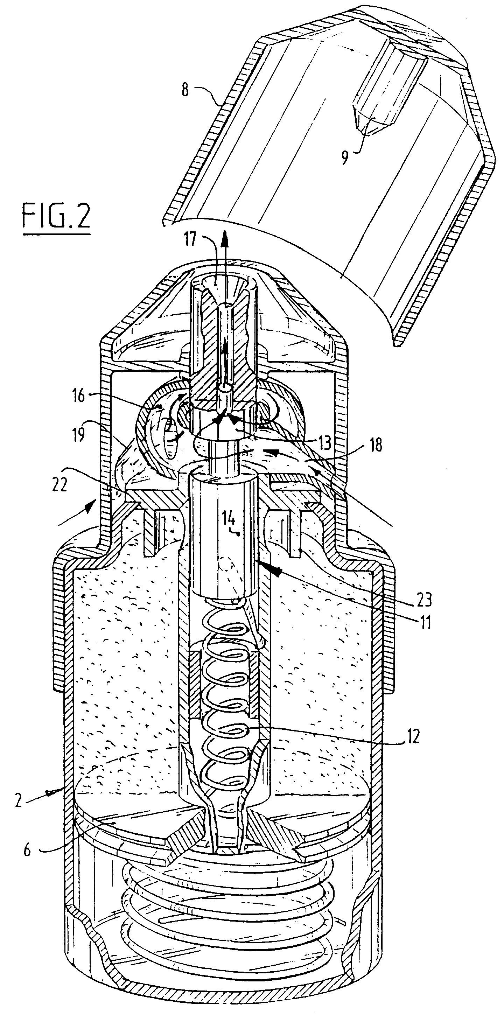

As will be apparent particularly from fig. 2, when the closing cap 8 is removed, a plunger 11 is moved axially with respect to the reservoir upward by means of the spring force of a coil spring 12. The plunger 11 is provided with a recess between a top piece 13 and a bottom piece 14, wherewith a dose of powder can be transported in the desired dosage from the reservoir to a mixing space 16. As is indicated with the arrows, when the opening 17 is sucked on, suction takes place through three channels, of which the channels 18 and 19 are visible in the figure, and via whirling of the air a good mixing of the powder and the air is obtained in the desired manner.

In preference the pin portion 9 of the closing cap 8 is provided with a tapering portion which fits into the conical opening 17 so that when the closing cap 8 is arranged the up and downward movable plunger 11 provided with a central channel 21 and the recess can be moved into the rest position shown in fig.1.

For guiding the powder to the recess in the plunger the housing 2 is preferably provided with an upper piece 22 provided with collar-like portion 23 which protrudes into the powder reservoir.

So that the powder-form material is carried well by the plunger, the latter is provided with a protrusion guided in a groove 24 that is arranged in the cylinder-shaped guiding 25 for the plunger, wherein this groove 24 causes the plunger to rotate slightly.

In a further embodiment of the present invention (not shown), a shaft 40 provided with wing-like elements 41 can be included at the centre line of the device shown in fig. 1-3. Preferably a rotating mechanism is provided such that for every dose shaft 40 is rotated and powder is stirred by elements 41 whereby the flow of the powder is improved. The rotating mechanism can correspond to such mechanism as used in a ballpoint-pen for rotating the writing element thereof.

The present inhaler device is suitable for giving a large number of doses and therefore for prolonged use. It can be manufactured from a comparatively small number of components, preferably of plastic.

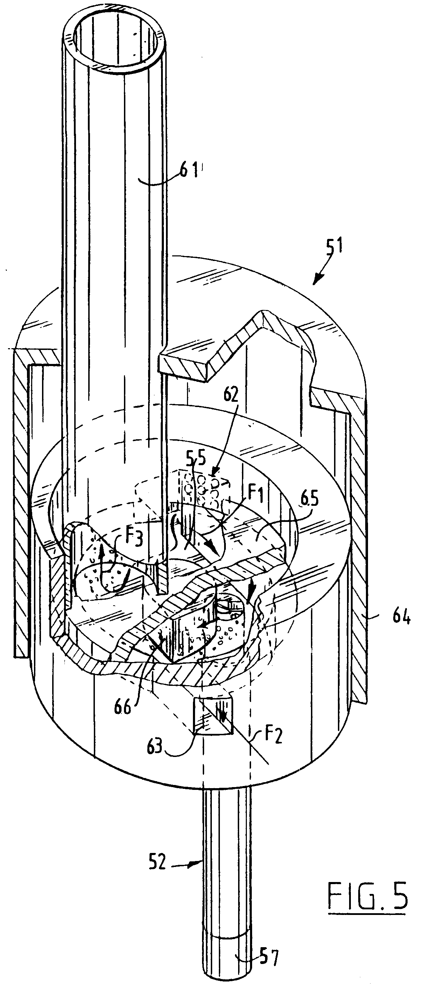

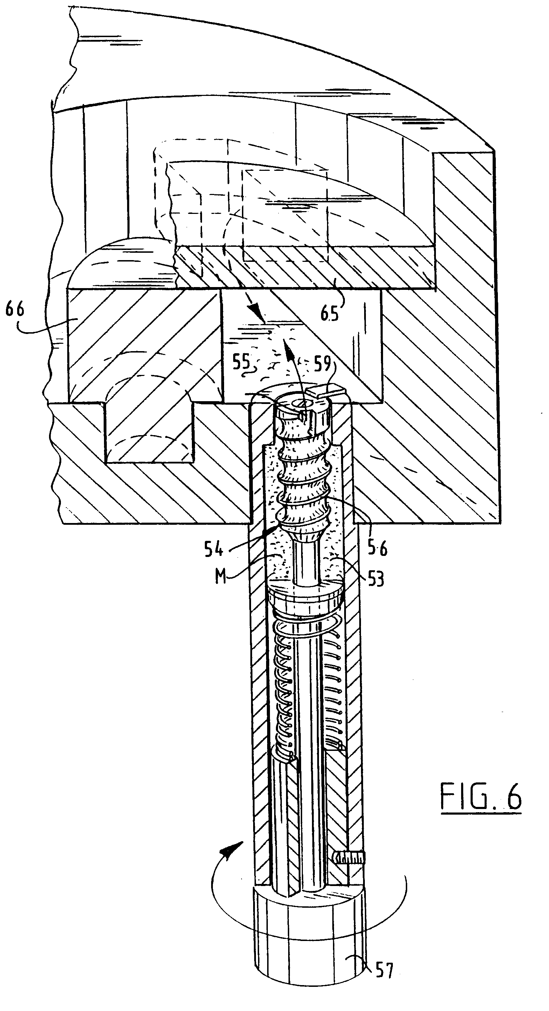

An inhaler device 51 (fig.5) is provided with a part 52 (fig.5, fig.6) in which is received a reservoir 53 for medium for inhaling in addition to a transporting mechanism 54 for transporting the medium out of the reservoir to a whirl chamber 55. The material for inhaling comprises carrier particles which are of minor importance in the effect on the bronchial tubes of a patient. These carrier particles are necessary however in order to prevent the medium M for inhaling from coagulating and thereby becoming barely transportable.

For transporting the medium M in doses of precise size a screw or worm 56 is preferably used which can be operated using a rotating knob 57. It has been found in tests that such a transporting mechanism is capable of transporting a medium M with an accuracy of less than 5% out of the reservoir 53 into the whirl chamber 55 at each revolution of the opening 58 away from and up to a closing member 59 for this opening.

The inhaler device is provided with an inhaling piece 61 for sucking in air via the nose or mouth of a patient along two feed channels 62 and 63 which are arranged in a peripheral wall 64 of the inhaler device. A flow of air, as designated with arrows F₁, carries the medium brought into the whirl chamber 55 under cover plate 65 along a guide means 66 and then collides with the air flow designated with arrow F₂ which is drawn in along feed channel 63. With a comparatively small suction force great air speeds occur here, in addition to whirls when these air flows F₁ and F₂ collide with one another. Shear forces hereby occur which separate the particles with medicinal effect from the carrier particles which usually have a greater diameter than that of the medicinal particles.

The member 66 for guiding the air (fig.7A) is provided on the side facing the feed channel 62 with a rounded form, while the side facing the feed channel 63 is sharp-edged and the angle is formed by flat wall porions 67 and 68 so that the air flow F₂ collides with the air flow F₁ transversely.

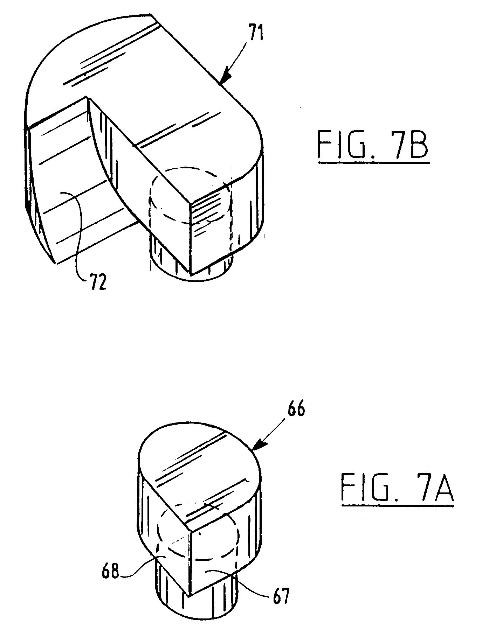

In a further modification (not shown), the inhaler device is provided with a guide means 71 for guiding the air (fig.7B) partially similar to the guide means 66, but which is additionally provided with an air guide portion 72 rising in the direction of the inhaling piece 11 and preventing unwanted air, as indicated with arrow F₃ in fig. 1, on the side of the guide means 71 remote from the doses of medium for inhaling from passing into the inhaling piece 11. An inhaler device provided with such a guide means 71 therefore requires an even smaller suction force, which is particularly important in the case of children and older patients with lung disorders.

The inhaler device according to the shown and described embodiment has, among others, the following advantages:
- the inhaler device is compact of structure and has a size such that it can easily be held in the hand;
- the inhaler device can be used in any desired position, with the inhaling piece horizontal or oriented upward or downward;
- the inhaler device requires little suction force, while great shear forces are applied to the medium or powder so that the medicinal particles are released from the carrier particles.

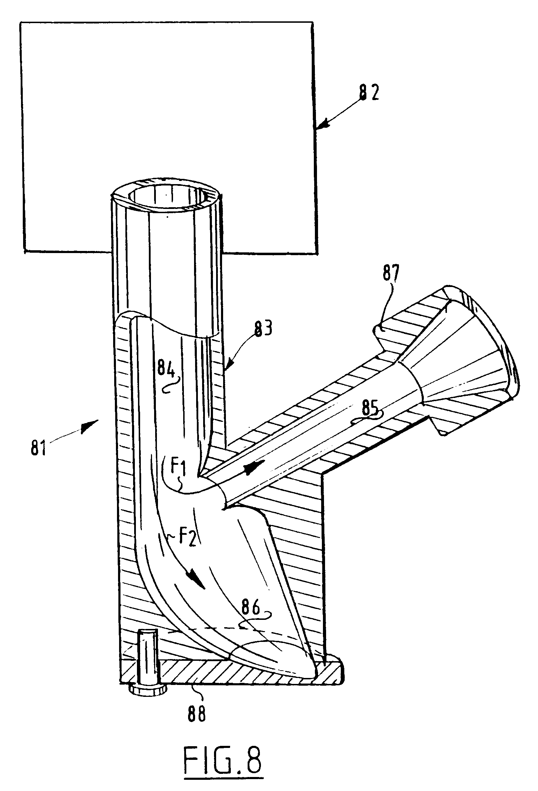

An inhaler device 81 (fig.8) comprises a schematically designated mixing chamber 82 in which a dosage for inhaling is mixed with air drawn into the mixing chamber 82 in a manner not shown. The inhaler device 81 can further be provided with a reservoir in which is received a supply of medium for inhaling, in addition to transporting means, likewise not shown, for transporting one dose of medium at a time out of this reservoir into the mixing chamber.

Coupled to the mixing chamber 82 is a suction piece 83 comprising an inlet channel 84, an outlet channel 85 and a collecting chamber 86. The inhaling piece 87 is sucked on by the patient via nose or mouth. A fraction of the aerosol, designated with arrow B₂ whereof the particles have a comparatively large diameter, for example greater than 10, 7, 5 or 1 µm, does not however reach the outlet channel 85 of the mass of the particles. These particles from the fraction B₂ are collected at the bottom of the collecting chamber 86 which must be emptied from time to time by releasing the cover 88.

With the inhaler device shown in fig.8, the angle enclosed by the centre lines of the outlet channel 85 and the inlet channel 84 has a value of approximately 60°, that is, the change of direction amounts to roughly 120°. The inlet channel is initially envisaged as having a diameter of approximately 1 cm, the outlet channel as having a diameter of 0.8 cm and the inlet channel as having a length of about 5 cm. The device is preferably realized in plastic. The figure is drawn to scale so that other proposed dimensions can be derived therefrom.

The present invention is not limited to the preferred embodiment shown and described. The rights are determined by the claims following hereinafter.

## Claims

1. A device (1) for inhaling an aerosol, comprising:
- a housing (2) in which a reservoir for powder (3) is received;
- an inhaling piece (7) on the housing; and
- transporting means for transporting the powder (3) from the reservoir to the inhaling piece (7) in a dose required for the aerosol, said transporting means including a plunger (11) which is provided with a recess, and which is movable reciprocally counter to spring pressure, characterized by compressing means (4, 6) for holding the content of the reservoir in compressed state and in that said plunger (1) is movable axially relative to said reservoir, between a first axial position wherein said recess is inside said reservoir and a second axial position wherein said recess is positioned outside of said reservoir and is in communication with said inhaling piece (7).

2. An inhaler device (1) according to claim 1, wherein the plunger (11) is provided with a protrusion for causing the plunger to rotate relative to the housing.

3. An inhaler device according to claim 1 or 2, provided with a closing cap (8) on the inhaler device, wherein a dose is transported to the inhaling piece (7) when the closing cap (8) is removed from the inhaling piece (7).

4. An inhaler device according claim 1, 2 or 3, provided with a whirl chamber (55, 82) wherein a first airflow drawn in through a first opening flows along the dose of medium for inhaling, collides with a second airflow drawn in through a second opening, so that whirling takes place whereby active constituents of the medium are released to a sufficient extent such as to have sufficiently small diameter.

## Patentansprüche

1. Vorrichtung (1) zum Inhalieren eines Aerosols, mit
- einem Gehäuse (2), in dem ein Reservoir für Pulver (3) aufgenommen ist;
- einem Inhalieraufsatz (7) auf dem Gehäuse;
- einer Transporteinrichtung zum Transportieren des Pulvers (3) aus dem Reservoir zu dem Inhalieraufsatz (7) in einer für das Aerosol benötigten Portion, wobei die Transporteinrichtung einen Kolben (11) aufweist, der mit einer Ausnehmung versehen ist und gegen Federkraft hin und her bewegbar ist, **gekennzeichnet** durch Kompressionsmittel (4,6), um den Inhalt des Reservoirs in komprimiertem Zustand zu halten, und daß der Kolben (1) axial relativ zu dem Reservoir bewegbar ist zwischen einer ersten axialen Position, in der sich die Ausnehmung innerhalb des Reservoirs befindet, und einer zweiten axialen Position, in der sich die Ausnehmung außerhalb des Reservoirs befindet und in Verbindung mit dem Inhalieraufsatz (7) steht.

2. Inhaliervorrichtung (1) nach Anspruch 1, bei der der Kolben (1) mit einem Vorsprung versehen ist, um eine Drehung des Kolbens relativ zum Gehäuse zu bewirken.

3. Inhaliervorrichtung nach Anspruch 1 oder 2, die mit einer Verschlußkappe (8) auf dem Inhalieraufsatz vesehen ist, wobei eine Portion zu dem Inhalieraufsatz (7) transportiert wird, wenn die Verschlußkappe (8) von dem Inhalieraufsatz (7) abgenommen wird.

4. Inhaliervorrichtung nach Anspruch 1, 2 oder 3, die mit einer Wirbelkammer (55,82) versehen ist, wobei eine durch eine erste Öffnung eingesaugte Luftströmung an der Portion von Inhaliermittel entlang strömt und mit einem durch eine zweite Öffnung eingesaugten zweiten Luftstrom kollidiert, so daß eine Verwirbelung stattfindet, wodurch aktive Bestandteile des Mittels in einem ausreichenden Ausmaß freigesetzt werden derart, daß sie einen ausreichend kleinen Durchmesser haben.

## Revendications

1. Appareil (1) d'inhalation d'un aérosol, comprenant :
- un boîtier (2) dans lequel est logé un réservoir de poudre (3),
- une pièce d'inhalation (7) placée sur le boîtier, et
- un dispositif de transport de la poudre (3) du réservoir à la pièce d'inhalation (7) selon une dose nécessaire pour l'aérosol, le dispositif de transport comprenant un piston (11) qui a une cavité et qui est mobile alternativement contre une pression d'un ressort antagoniste, caractérisé par un dispositif de compression (4, 6) destiné à maintenir le contenu du réservoir dans un état comprimé, et en ce que le piston (1) est mobile axialement par rapport au réservoir, entre une première position axiale dans laquelle la cavité se trouve à l'intérieur du réservoir et une seconde position axiale dans laquelle la cavité se trouve à l'extérieur du réservoir et en communication avec la pièce d'inhalation (7).

2. Appareil d'inhalation (1) selon la revendication 1, dans lequel le piston (11) a une saillie destinée à provoquer la rotation du piston par rapport au boîtier.

3. Appareil d'inhalation selon la revendication 1 ou 2, ayant un capuchon de fermeture (8) de l'appareil d'inhalation, dans lequel une dose est transportée vers la pièce d'inhalation (7) lorsque le capuchon de fermeture (8) est retiré de la pièce d'inhalation (7).

4. Appareil d'inhalation selon la revendication 1, 2 ou 3, ayant une chambre de tourbillonnement (55, 82) dans laquelle un premier courant d'air aspiré au travers d'une première ouverture s'écoule avec une dose du milieu à inhaler, et rencontre un second courant d'air aspiré au travers d'une seconde ouverture si bien qu'un tourbillonnement s'effectue de sorte que des constituants actifs du milieu sont libérés en quantité suffisante pour qu'ils aient un diamètre suffisamment petit.
